# EUROPÄISCHE PATENTANMELDUNG

(11) **EP 4 480 575 A1**
(43) Veröffentlichungstag der Anmeldung: **25.12.2024**
(21) Anmeldenummer: 23181210.8
(22) Anmeldetag: 23.06.2023
(51) Int. Cl.: B01J 8/02, B01J 8/06, C01C 1/04

(54) **GEKÜHLTER REAKTOR ZUM DURCHFÜHREN EXOTHERMER GLEICHGEWICHTSREAKTIONEN**

(71) Anmelder: L'AIR LIQUIDE, SOCIETE ANONYME POUR L'ETUDE ET L'EXPLOITATION DES PROCEDES GEORGES CLAUDE, 75007 Paris (FR)
(72) Erfinder: Castillo-Welter, Frank, 60388 Frankfurt am Main (DE); Do, Nga Thi Quynh, 60388 Frankfurt am Main (DE); Haag, Stéphane, 60388 Frankfurt am Main (DE)
(74) Vertreter: Air Liquide

(57) **Zusammenfassung**

Es wird ein Reaktor zum Durchführen exothermer Gleichgewichtsreaktionen, insbesondere für die Durchführung der Methanolsynthese und/oder Ammoniaksynthese, durch heterogen-katalysierte Umsetzung der entsprechenden Eduktgase vorgeschlagen, der es ermöglicht, die Einstellung des Reaktionsgleichgewichts im Reaktor zu überwinden. Dazu wird erfindungsgemäß durch Unterkühlen des Kühlmittels die Kühlmitteltemperatur entlang der Längskoordinate des Reaktors nachgeführt und somit optimiert.

## Beschreibung

### Gebiet der Erfindung

Die Erfindung betrifft einen Reaktor zum Durchführen exothermer Gleichgewichtsreaktionen, bei denen ein gasförmiges Einsatzgemisch an einem festen Katalysator mindestens teilweise zu einem Produktgemisch umgesetzt wird. Anwendungsbeispiele sind die Durchführung der Methanolsynthese durch Umsetzung von Wasserstoff und Kohlenoxide umfassendem Synthesegas sowie die Durchführung der Ammoniaksynthese durch Umsetzung von Wasserstoff und Stickstoff umfassendem Einsatzgas, jeweils als heterogen-katalysierte Reaktion an festen Katalysatoren.

### Stand der Technik

Reaktoren zur Durchführung exothermer Gleichgewichtsreaktionen sind der Fachwelt seit langem bekannt. Eine industriell besonders wichtige Reaktion dieses Typs ist beispielsweise die Methanolsynthese durch heterogen-katalytische Umsetzung von Synthesegas, also Gemischen von Wasserstoff und Kohlenoxiden. In Ullmann's Encyclopedia of Industrial Chemistry, Sixth Edition, 1998 Electronic Release, Kapitel "Methanol", Unterkapitel 5.2 "Synthesis" werden verschiedene Grundverfahren zur Herstellung von Methanol durch katalytische Umsetzung von Wasserstoff und Kohlenstoffoxide enthaltendem Synthesegas beschrieben, bei denen solche Reaktoren eingesetzt werden.

Eine weitere, industriell besonders wichtige exotherme Gleichgewichtsreaktion ist die Ammoniaksynthese, die in Ullmann's Encyclopedia of Industrial Chemistry, Sixth Edition, 1998 Electronic Release, Kapitel "Ammonia", Unterkapitel 4 "Production" ausführlich erläutert wird.

Ein moderneres, zweistufiges Verfahren zur Herstellung von Methanol ist beispielsweise aus der europäischen Patentschrift EP 0 790 226 B1 bekannt. Das Methanol wird in einem Kreisprozess hergestellt, bei dem eine Mischung von frischem und teilweise reagiertem Synthesegas zunächst einem wassergekühlten Reaktor und dann einem gasgekühlten Reaktor zugeführt wird, in welchen das Synthesegas jeweils an einem kupferbasierten Katalysator zu Methanol umgesetzt wird. Das in dem Prozess hergestellte Methanol wird von dem zurückzuführenden Synthesegas abgeschieden, welches dann als Kühlmittel im Gegenstrom durch den gasgekühlten Reaktor hindurchgeführt und auf eine Temperatur von 220 bis 280°C vorgewärmt wird, bevor es in den ersten Synthesereaktor eingebracht wird. Ein Teil des zurückzuführenden Synthesegases wird als Spülstrom (sog. Purge) aus dem Verfahren entfernt, um zu verhindern, dass sich Inertkomponenten innerhalb des Synthesekreislaufes anreichern. Diese Maßnahme wird auch in der deutschen Offenlegungsschrift DE 2934332 A1 und der europäischen Patentanmeldung EP 1016643 A1 gelehrt.

Als Inertkomponente im Sinne der Methanolsynthese und auch der Ammoniaksynthese wird dabei nicht umgesetztes Methan aus der Synthesegaserzeugung betrachtet, da sich dieses unter den Bedingungen der Methanol- bzw. Ammoniaksynthese ebenfalls nicht weiter umsetzt. Dasselbe gilt für Argon, das über Feedströme in die Synthesegaserzeugung gelangt.

In der wassergekühlten Reaktorstufe wird üblicherweise der Hauptumsatz des Synthesegases (CO, CO₂, H₂) erzielt und der größte Teil der Reaktionswärme abgeführt, während in der gasgekühlten Stufe bei milderen Bedingungen ein dennoch erheblicher Teil des Synthesegases umgesetzt wird.

Bei dem wassergekühlten Reaktor (WCR) handelt es sich üblicherweise um einen Röhrenreaktor mit entsprechenden Rohrendplatten, bei dem der Katalysator in die Rohre gefüllt wird, während die Kühlung mittels siedendem Wasser bzw. Dampferzeugung auf der Mantelseite um die Rohre erfolgt (Innenraum). Im gasgekühlten Reaktor (GCR) erfolgt die Kühlung mit dem Einsatzgas, welches durch die Rohre geführt wird und sich auf dem Weg zur ersten Reaktionsstufe (WCR) erwärmt, während der Katalysator um die Rohre gefüllt wird und die Reaktion auf der Mantelseite des GCR stattfindet. Die Reaktionsstufen sind hinsichtlich ihrer Nennweite mit großen bzw. sehr großen Rohrleitungen verbunden; je nach Anlagenkapazität sind Rohrdurchmesser von bis zu 1 m möglich. Dies ist vor allem auf die großen Gasmengen zurückzuführen, die zu der zweiten Stufe zurückgeführt (Recycle-Gas) und dem Frischgas, also frischem Synthesegas aus der Gaserzeugung, zugemischt werden. Das sich ergebende Gasgemisch aus Rückführungsgas und Frischgas wird nach erfolgter Vorwärmung im GCR der ersten Reaktionsstufe (WCR) zugeführt. Die Rückführungsgasmenge (Recycle-Gas) ist üblicherweise deutlich größer als die Frischgasmenge und ist vom erzielten Umsatz in der Reaktorsektion abhängig. Das Rückführverhältnis RR (RR=R/F) aus Rückführungsgasmenge (R) zu Frischgasmenge (F) liegt oft über 2 und in einigen Fällen sogar über 3,5. Je niedriger der Umsatz von Synthesegas durch die Reaktorsektion per Durchgang ist, um so höher ist das erforderliche Rückführverhältnis RR, um eine hinreichende Ausbeute zu erzielen.

Damit erhöht sich die umlaufende Gasmenge entsprechend, was die Belastung der Reaktoren erhöht und größere Rohrnennweiten der verbindenden Rohrleitungen bedarf und auch zu einem höherem Bedarf an Kompressionsenergie (höhere Durchflussmenge und Druckverlust) führt.

Ein aktuelles Verfahren zur Ammoniaksynthese wird beispielsweise in der Patentveröffentlichung WO 2002/038499 A1 beschrieben. Im Vergleich zu dem für die Methanolsynthese verwendeten Synthesegas ist es dabei wichtig, bei dem Synthesegas für die Ammoniaksynthese den Anteil an Kohlenoxiden vollständig zu eliminieren, so dass Wasserstoff als allein verbliebener Synthesegasbestandteil in die Ammoniaksynthese gelangt. Dies erfolgt zunächst durch die Konvertierung des im Synthesegas enthaltenen Kohlenmonoxids (CO-Konvertierung), einer sich an diese anschließende Kohlendioxidentfernung mittels eines Sorptionsverfahrens und schließlich mittels kryogener Gaszerlegung.

Nachteilig bei den genannten Synthesereaktionen ist es, dass sie als exotherme Gleichgewichtsreaktionen durch das Reaktionsgleichgewicht limitiert werden. Wünschenswert wäre es daher, Reaktoren und Verfahren zu ihrem Betrieb anzugeben, mit denen diese Limitierungen überwunden werden können.

### Beschreibung der Erfindung

Die Aufgabe der vorliegenden Erfindung besteht daher darin, einen Reaktor und eine Verfahren zu dessen Betrieb anzugeben, der die beschriebenen Nachteile der aus dem Stand der Technik bekannten Reaktoren nicht aufweist und der insbesondere einen hohen Umsatz bezüglich der Zielprodukte der exothermen Gleichgewichtsreaktion pro Reaktordurchgang sowie die Möglichkeit bietet, die Reaktionsbedingungen entlang der Längskoordinate des Reaktors nachzuführen und somit zu optimieren, was beispielsweise im Falle der Methanolsynthese zu einer Reduzierung des Rückführverhältnisses auf kleinere Werte führt, wie sie bei Verwendung der aus dem Stand der Technik bekannten Reaktoren bekannt sind.

Diese Aufgabe wird in einem ersten Aspekt durch einen Reaktor mit den Merkmalen des Anspruchs 1 und in einem weiteren Aspekt durch ein Verfahren mit den Merkmalen des Anspruchs 8 gelöst. Weitere Ausgestaltungen gemäß weiterer Aspekte der Erfindung ergeben sich aus den Unteransprüchen der jeweiligen Kategorie.

Unter Fluidverbindung zwischen zwei Bereichen des erfindungsgemäßen Reaktors wird dabei jegliche Art von Verbindung verstanden, die es ermöglicht, dass ein Fluid, beispielsweise der Einsatzgasstrom oder der Synthesegasproduktstrom, von dem einen zu dem anderen der beiden Bereiche strömen kann, unbeachtlich etwaiger zwischengeschalteter Bereiche oder Bauteile.

Unter Mitteln zum Einleiten, Ausleiten usw. werden alle die Vorrichtungen, Vorrichtungsbestandteile, Baugruppen und Bauteile verstanden, die es ermöglichen, dass das betreffende Fluid den betrachteten räumlichen Bereich, beispielsweise einen Behälter, verlässt. Hierunter werden insbesondere Rohrleitungen, Pumpen, Verdichter, sonstige Fördervorrichtungen sowie die entsprechenden Durchtrittsöffnungen in der Behälterwand verstanden.

Unter der katalytischen Aktivität, insbesondere im Zusammenhang mit einer unterschiedlichen katalytischen Aktivität beim Vergleich zweier unterschiedlicher Katalysatoren, wird der erzielte Umsatzgrad pro Längeneinheit des Katalysatorbetts von Edukten zu Produkten verstanden. Die Aktivität wird beeinflusst von der chemischen Zusammensetzung, Dotierung, Vergiftung, verfügbaren Oberfläche etc. des Katalysatormaterials, aber auch durch die Geometrie der Katalysatorpartikel und texturellen Parametern des Katalysatorbetts, z. B. dessen Porosität oder Packungsdichte. Aufgrund der Exothermie der betrachteten Reaktionen korreliert eine hohe katalytische Aktivität mit einer hohen Wärmefreisetzung pro Längeneinheit des Katalysatorbetts.

Als Rohrreaktor im Sinne der vorliegenden Beschreibung wird ein Reaktionsapparat verstanden, der ein oder eine Vielzahl von Rohren mit in der Regel kreisförmigem Querschnitt umfasst, das oder die mit Schüttungen eines oder mehrerer Katalysatoren gefüllt sind, wobei das oder die Rohre in der Regel aus einem metallischen Werkstoff gefertigt sind. Der Eduktstrom tritt an der einen offenen Rohrseite in den Rohrreaktor ein, durchströmt ihn kontinuierlich und wird dabei zu einem Produktstrom umgesetzt, der an der gegenüberliegenden offenen Rohrseite kontinuierlich abgezogen wird. Eine axiale Rückvermischung der durch den Rohrreaktor strömenden Stoffströme findet dabei nicht oder in nur geringem Ausmaß statt. Bei exothermen Reaktionen bildet sich daher in Abhängigkeit der Wärmetönung der Reaktion ein axiales Temperaturprofil mit mehr oder weniger ausgeprägten Maxima aus (sog. "hot spots"). Der mit dem Temperaturprofil durchlaufene Temperaturbereich wird im Sinne dieser Beschreibung als Reaktionstemperaturbereich verstanden.

Als Plattenreaktor wird ein Reaktionsapparat verstanden, bei dem eine Katalysatorschüttung bzw. ein Katalysatorbett eines festen, partikelförmigen Katalysators zwischen zwei in der Regel parallel zueinander ausgerichteten Platten angeordnet ist. Diejenigen Seiten dieser Anordnung, die nicht dem Einleiten von Edukten bzw. dem Ausleiten von Reaktionsprodukten dienen, sind in der Regel an ihren Rändern verschlossen, beispielsweise durch fluiddichten Kontakt mit der Innenwand des Mantelrohrs des Reaktors oder durch entsprechende Verschweissungen.

Als Plattenreaktor wird auch ein Reaktionsapparat verstanden, bei dem das Katalysatorbett nicht zwischen zwei ebenen Platten, sondern zwischen zwei Thermoblechen angeordnet ist. Ein Thermoblech im Sinne der Erfindung besteht aus zwei Blechen, welche an den Rändern verbunden, vorzugsweise zusammengeschweißt sind und über deren Oberfläche eine Vielzahl von zusätzlichen Verbindungen, vorzugsweise Punktschweißungen, die die Platten ebenfalls miteinander verbinden, verteilt sind. Solche Platten können automatisiert von Robotern oder Maschinen und somit zu sehr günstigen Preisen hergestellt werden. Nach der Verschweißung werden die Bleche durch eine hydraulische Umformung, in der Regel das Einpressen einer Flüssigkeit unter hohem Druck, expandiert, wodurch kissenartige Kanäle zwischen den Blechen entstehen, durch die ein Heiz- oder Kühlfluid geleitet werden kann. Über die Wärmetransporträume kann daher bestimmten Bereichen des Reaktor durch das Durchleiten von Heiz- bzw. Kühlfluiden sowohl Wärmeenergie zu- als auch abgeführt werden.

Die Reaktionszonen können bei Verwendung von Thermoblechen so ausgestaltet werden, dass in dem Reaktor zunächst zwei Thermobleche im Wesentlichen parallel angeordnet werden. Im Wesentlichen parallel im Sinne der Erfindung bedeutet, dass die Ausrichtung der Thermobleche zueinander von der Parallelen maximal um +/- 20°, bevorzugt maximal um +/- 10°, besonders bevorzugt maximal um +/- 5°, ganz besonders bevorzugt maximal um +/- 2° voneinander abweicht. Danach kann der Freiraum zwischen den Thermoblechen mit einer Schüttung eines festen, körnigen, partikelförmigen oder pelletförmigen Katalysators aufgefüllt werden, wobei der seitliche Abschluss des sich ergebenden Katalysatorbettes durch Netze, Gitter, Lochbleche, Roste, Schüttungen von Inertmaterial und/oder die Reaktorinnenwand gebildet wird.

Besonders bevorzugt schließen sich parallel zu dieser Anordnung mindestens ein, bevorzugt mehrere weitere, beabstandete Thermobleche an, wobei sich insgesamt ein Plattenpaket ergibt und die Freiräume zwischen den Thermoblechen mit Katalysatorschüttungen aufgefüllt werden. Auf diese Weise wird in der Reaktionszone eine kompakte, sandwichartige Struktur mit einer intensiv wirkendenden, sich über die Länge der Reaktionszone erstreckenden Kühlvorrichtung erhalten. Die Beaufschlagung der einzelnen Katalysatorschüttungen mit dem Reaktionsgasgemisch erfolgt dabei parallel. Die Plattenpakete können, bezogen auf die mit Katalysator gefüllten Freiräume, parallel oder senkrecht zur Längsachse des Reaktors ausgerichtet werden.

Die Abstände zwischen den Thermoplatten können gemäß der Wärmetönung der durchzuführenden Reaktion ausgewählt werden: Für stark exotherme Reaktionen wird dieser Abstand kleiner gewählt als für schwächer exotherme Reaktionen. Dabei werden in der ersten Reaktionszone kleinere Plattenabstände bevorzugt, da hier der größte Umsatz erzielt wird und die größte Wärmeabfuhr realisiert werden muss. Die Thermoblechplattenabstände der ersten Reaktionszone betragen im Falle der Methanolsynthese bevorzugt 20 bis 45 mm. Der Abstand bezieht sich dabei auf den Abstand von Mittellinie zur Mittellinie, d. h. der lichte Abstand zwischen den Platten fällt je nach Thermoblechdicke und Expansion des Hohlraumes entsprechend kleiner aus. Ferner wird der Abstand an die Abmessungen der Katalysatorpartikel angepasst, um eine optimale Wärmeabfuhr sowie ein gutes Schüttgutverhalten beim Befüllen und Entleeren des Katalysators ohne Brückenbildung sicherzustellen. In der zweiten und den nachfolgenden Reaktionszonen werden die Abstände üblicherweise größer gewählt.

Gegenüber Rohrreaktoren weisen Plattenreaktoren den Vorteil auf, dass in der Regel pro Volumeneinheit des Katalysators eine größere Kühlfläche zur Verfügung steht.

Als Längsachse wird in der vorliegenden Beschreibung jene Achse eines Apparates oder einer Vorrichtung verstanden, die der Richtung seiner größten Ausdehnung entspricht. Im Sinne der vorliegenden Beschreibung wird eine Anordnung eines Apparates oder einer Vorrichtung, bei der ihre Längsachse parallel zu der mittels der Schwerkraft vermittelten Senkrechte angeordnet ist, als stehende Anordnung verstanden.

Als Kühlmittel werden bei der erfindungsgemäßen Reaktorkühlung bevorzugt Medien eingesetzt, die sich in dem Reaktionstemperaturbereich bei dem Reaktionsdruck in der Nähe ihres Siedepunktes befinden und daher leicht verdampfen. Dies sichert eine gute Wärmeabfuhr aus dem Katalysatorbett bei der exothermen Reaktion durch den guten Wärmeübergang auf der Seite des verdampfenden Mediums und erlaubt eine präzise Temperaturregelung über den Druck. Um unterschiedliche Temperaturbedingungen in den verschiedenen Stufen einzustellen, wird der Druck auf der Seite des Kühlmediums im Innenraum geregelt. Mit zunehmender Katalysatorlaufzeit können die Bedingungen mittels entsprechender Einstellung des Druckes im Innenraum angepasst werden und somit die Reaktionstemperatur nachgeführt werden, um beispielsweise den Umsatz entsprechend hoch zu halten.

Bezüglich der gewünschten Reaktionsbedingungen kann beispielsweise bei der Methanolsynthese Wasser bzw. Wasserdampf als Wärmeträger eingesetzt werden. Jedoch zeigt sich, dass bei Verwendung von Wasser für den gewünschten Temperaturbereich relativ große Druckunterschiede eingestellt werden müssen, um einen breiten Temperaturbereich abzudecken (z. B. 250 °C: ca. 40 bar, 264 °C: ca. 50 bar). Benutzt man dagegen ein verdampfendes Wärmeträgeröl (z. B. Dowtherm A) in einem Kreislauf zur Wasserdampferzeugung, so kann man in einem sehr engen Druckbereich arbeiten und dennoch einen großen Temperaturbereich abdecken (Bsp.: 255 °C: 0,97 bar, 305 °C: 2,60 bar, entsprechend einem Temperaturbereich von 50 °C bei einem Druckunterschied von nur 1,6 bar. Dadurch kann man mit einer einfachen Wärmeträgeröl-Dampftrommel auf dem entsprechendem Anlagenniveau (ca. 20 bis 25 m) arbeiten und allein die geodätische Zulaufhöhe nutzen, um die individuellen Druck- bzw. Temperaturbereiche einzustellen.

An die Kühlmittel-Kühlvorrichtung wird im Sinne dieser Beschreibung die Anforderung gestellt, dass sie in der Lage ist, im Zusammenwirken mit der Druckregelvorrichtung eine definierte Kühlmitteltemperatur unterhalb des Siedepunktes des Kühlmittels bei dem Druck im Innenraum einzustellen (Unterkühlung). Als Innenraum wird der freie, von dem Kühlmittel durchströmbare Raum zwischen der Außenwand des mindestens einen Rohrreaktors oder den Außenwänden des mindestens einen Plattenreaktors und der Innenwand des Mantelrohrs verstanden.

Unter den Bedingungen einer exothermen Gleichgewichtsreaktion im Allgemeinen, und spezifisch den Methanolsynthesebedingungen bzw. Ammoniaksynthesebedingungen sind die dem Fachmann an sich bekannten Verfahrensbedingungen, insbesondere von Temperatur, Druck, Verweilzeit, zu verstehen, wie sie oben beispielhaft genannt und detailliert im einschlägigen Schrifttum erörtert werden und bei denen mindestens ein Teilumsatz, bevorzugt allerdings technisch relevante Umsätze der Edukte zu den Produkten des jeweiligen Verfahrens erfolgt. Dasselbe gilt für die Auswahl eines geeigneten Katalysators und geeigneter Betriebsbedingungen desselben, da im Sinne der vorliegenden Erfindung beide genannten Verfahren heterogen-katalytisch betrieben werden, Entsprechende Methanolsynthesereaktoren und Ammoniaksynthesereaktoren sind der Fachwelt an sich bekannt und beispielsweise in dem eingangs erwähnten Schrifttum beschrieben.

Der Erfindung liegt die Erkenntnis zugrunde, dass durch eine optimale Temperaturführung insbesondere in axialer Richtung des Reaktors die Produktionsraten bzw. Raum-Zeit-Ausbeuten entlang des Reaktionsweges deutlich verbessert werden können. Das Temperaturprofil entlang des Reaktionsweges wird durch den Einsatz des erfindungsgemäßen Kühlkonzeptes erheblich verbessert, wodurch ein deutlich höherer Umsatz per Durchgang erzielt wird. Die Reaktorkühlung gemäß der Erfindung ist für katalysatorgefüllte Rohrreaktoren als auch für katalysatorgefüllte Plattenreaktoren anwendbar.

Auch die Nebenproduktbildung bei der Methanolsynthese verringert sich bei Verwendung des erfindungsgemäßen Reaktors gegenüber dem Stand der Technik.

Ein verbessertes Temperaturprofil im Reaktor lässt sich grundsätzlich auch mit Hilfe eines Katalysator-Layer-Managements erzielen. Dabei würde man in dem Bereich, in dem man den höchsten Umsatz (Exothermie) und damit die höchsten Temperaturen erwartet, einen weniger aktiven Katalysator einsetzen und in Bereichen, wo weniger Umsatz erwartet wird, einen aktiveren Katalysator einsetzen. Jedoch ist ein solches Katalysator-Layer-Management relativ unflexibel, da man die verschiedenen Katalysatorlagen auf Basis einer bestimmten Katalysatoraktivität und einer entsprechenden Gaszusammensetzung auswählen und festlegen muss. Die Katalysatoraktivität verändert sich jedoch über seine Laufzeit der Syntheseanlage durch seine fortschreitende Desaktivierung.

Das Layer-Management und die zugehörige Kühlung des Reaktionsbettes müssen aufeinander abgestimmt werden. Während der Katalysatorlaufzeit und der damit verbundenen Katalysatordesaktivierung ändern sich die Bedingungen und eine Anpassung der Reaktionstemperatur und der zugehörigen Kühlung/Kühltemperatur ist wünschenswert, um die Desaktivierung zumindest teilweise zu kompensieren und einen hohen Umsatz bei geringer Nebenproduktbildung sicherzustellen. Mit den aus dem Stand der Technik bekannten Reaktoren lässt sich eine Anpassung der Kühlung nur für den gesamten Reaktor vornehmen; üblicherweise desaktivieren aber nicht alle Katalysatorschichten über die Betriebszeit in gleichem Maße. Die Einstellung spezifischer Reaktionsbedingungen stellt daher immer einen Kompromiss dar.

Mit dem erfindungsgemäßen Ansatz können die Reaktionsbedingungen entlang der Längsachse des katalytischen Festbettreaktors individuell in Abhängigkeit von der Katalysatoraktivität, der Gaszusammensetzung in jeder Stufe, und einem etwaigen Vorhandensein mehrere Schichten unterschiedlicher Katalysatoren (Layer-Management), auch über die Laufzeit angepasst werden. Damit werden ein hoher Umsatz und eine geringe Nebenproduktbildung über den Gesamtreaktor erreicht.

Mit der optimierten Temperaturführung werden auch die Maximaltemperaturen (sowie Temperaturspitzen, sog. Hot-Spots) im Katalysatorbett reduziert. Dies wirkt sich neben der Ausschleusung des Koppelproduktes aus dem Reaktionssystem, beispielsweise von Wasser bei der Methanolsynthese, positiv auf die Katalysatorlaufzeit aus. Es ist bekannt, dass hohe Temperaturen im Katalysatorbett zu einer rascheren Katalysatordesaktivierung beitragen.

Ein Aspekt der Erfindung ist es, einen Reaktoraufbau mit einem verdampfenden Kühlmedium, beispielsweise Wasser oder Wärmeträgeröl, auf der Kühlseite des Reaktors zu verwenden. Dadurch kann ein sehr guter Wärmeübergang auf der Kühlseite in den Zonen gewährleistet werden, in denen das Kühlmedium teilweise verdampft. Bei stehender Anordnung des Reaktors erfolgt die Verdampfung bevorzugt im oberen Teil des Reaktors, wo der höchste Umsatz auf der Reaktionsseite stattfindet und eine entsprechende Kühlung erforderlich und sinnvoll ist. Daher wird das Kühlmedium flüssig und unterkühlt in den unteren Bereich der Kühlseite des Reaktors eingeführt, während das Reaktionsgas von oben in die Reaktionsseite eintritt. Im Prinzip strömt das Kühlmedium auf der Kühlseite (Innenraum) im Gegenstrom zum Reaktionsgas.

Das Kühlmedium wird in einem Beispiel durch natürliche Zirkulation umgewälzt, also durch Dichteunterschiede durch die Verdampfung und/oder Temperaturunterschiede entlang des Kühlkreislaufs, aber auch eine erzwungene Zirkulation könnte durch den Einsatz von Pumpen innerhalb des Kühlkreislaufs erfolgen, die die unterkühlte Flüssigkeit aus einer Dampftrommel als Kühlmedium-Reservoir zur Kühlseite des Reaktors pumpen würden.

Zur Optimierung der Reaktorleistung wird das Kühlmedium, das aus dem Reservoir im gesättigten Zustand bereitgestellt wird, vor dem Eintritt in die Kühlseite des Reaktors unterkühlt. Die verringerte Kühltemperatur führt zu niedrigeren Reaktionstemperaturen auf der Prozessseite im Reaktor, was im Hinblick auf das thermodynamische Gleichgewicht der exothermen Reaktion von Vorteil ist und schließlich die Umsatzraten verbessert. Niedrigere Temperaturen sind auch in anderer Hinsicht vorteilhaft (z. B. geringere Bildung von Nebenprodukten, verlängerte Lebensdauer des Katalysators).

Für den Reaktor können alle relevanten Reaktionsapparate verwendet werden, bei denen hauptsächlich eine Gegenströmung von Kühlmedium und Reaktionsgas realisiert werden kann. Beispiele hierfür sind Rohrreaktoren, Rohrbündelreaktoren und Plattenreaktoren.

Für die Unterkühlung des Kühlmediums können verschiedene Wärmetauschertypen eingesetzt werden. Wesentlich ist ein geringer Druckverlust auf der Seite des Kühlmediums, wenn eine natürliche Zirkulation angewendet wird. Um entsprechende Umwälzraten des Kühlmediums zu realisieren, kann in einem Beispiel die Flüssigkeitsförderhöhe aus dem Kühlreservoir im Hinblick auf den Gesamtdruckabfall im Kühlkreislauf ausgelegt werden.

Eine Möglichkeit, die gewünschte Unterkühlung zu erreichen, könnte ein Luftkühler sein, der im Fallrohr zwischen dem Kühlmittelreservoir und dem Einlass der Reaktorkühlseite installiert wird. Die Querschnittsfläche des Wärmetauschers für die Kühlmittelseite wird so ausgelegt, dass ein niedriger Druckabfall erreicht wird.

Zur Anpassung der Temperatur am Eintritt des Kühlmediums in die Kühlseite des Reaktors kann ein Bypass um den Wärmetauscher für die Unterkühlung verwendet werden, um den Durchfluss durch den Wärmetauscher zu verringern, was zu einer geringeren Unterkühlung führt. Im Bypass wird in einem Beispiel ein Regelventil installiert, um den gewünschten Durchfluss im Bypass bzw. im Wärmetauscher zu steuern. Optional könnte ein weiteres Ventil hinter dem Wärmetauscher eingebaut werden, um den Durchfluss durch den Wärmetauscher bei Bedarf auf ein Minimum zu drosseln. Dadurch wäre es möglich, den Wärmetauscher vollständig zu umgehen. Das Regelventil im Bypass wird in einem Beispiel von einem Temperaturregler gesteuert, der die Temperatur des Kühlmediums vor Eintritt in die Kühlseite des Reaktors regelt. In einem weiteren Beispiel könnte die gemessene Temperatur des Reaktorproduktgases auf der Prozessseite am Reaktorausgang verwendet werden, um das Bypassventil direkt oder indirekt über eine Kaskade auf dem oben erwähnten Temperaturregler für die Kühlung zu steuern.

Bei Verwendung eines Luftkühlers kann die Kühlung auch über die üblichen Steuergeräte (z.B. Drehzahlregelung, Lüfterflügelverstellung, Jalousie) gesteuert werden. Die Bypass-Lösung wäre jedoch für alle Arten von Wärmetauschern geeignet.

Für eine verbesserte Wärmeintegration ist gemäß eines Beispiels sinnvoll, anstelle eines Luftkühlers einen Wärmetauscher einzusetzen, der mit dem aus dem Reaktor ausgeleiteten Kühlmedium gekühlt wird. Dabei wird das Kühlmedium vorgewärmt, während auf der heißen Seite des Wärmetauschers das Kühlmedium des Reaktorkühlkreislaufs abgekühlt wird. Durch die Vorwärmung kann mehr Wärme aus dem System zurückgewonnen und die Dampfproduktion in der Dampftrommel erhöht werden.

Ein solcher Wärmetauscher könnte beispielsweise ein Doppelrohr-Wärmetauscher, ein Mehrrohr-Wärmetauscher, ein Platten-Wärmetauscher oder ein anderer Wärmetauschertyp sein. In jedem Fall ist ein Gegenstromsystem zwischen heißer und kalter Seite zu bevorzugen, um eine optimale Steuerung und Effizienz zu erreichen. Außerdem muss die Konstruktion des Wärmetauschers einen niedrigen Druckabfall auf der heißen Seite, die in den Kühlmittelkreislauf des Reaktorsystems eingebunden ist, gewährleisten. In einem Beispiel wird der Wärmetauscher in die Leitung zwischen dem Kühlmittelreservoir (Dampftrommel) und dem Einlass der Reaktorkühlseite integriert. Die Strömung auf der heißen Seite erfolgt bei stehender Anordnung vom oberen Ende des Wärmetauscher nach unten, während die Vorwärmung des Kühlmediums auf der kalten Seite vorzugsweise vom unteren Eingang nach oben erfolgt. Außerdem wird bei der Konstruktion des Wärmetauschers in einem Beispiel auf jeder Seite des Wärmetauschers nur ein Durchgang verwendet und Mehrfachdurchgänge vermieden, um eine gute Steuerbarkeit der Wärmeübertragungsleistung zu gewährleisten.

Mit der Erfindung wird ein Reaktor zum Durchführen exothermer Gleichgewichtsreaktionen, insbesondere für die Durchführung der Methanolsynthese und/oder der Ammoniaksynthese, durch heterogen-katalysierte Umsetzung von Synthesegas vorgeschlagen, der es ermöglicht, die Reaktionsbedingungen entlang der Längskoordinate des Reaktors nachzuführen und somit zu optimieren, was beispielsweise im Falle der Methanolsynthese zu einer Reduzierung des Rückführverhältnis auf kleinere Werte führt, wie sie bei Verwendung der aus dem Stand der Technik bekannten Reaktoren bekannt sind. Entsprechende Rückführleitungen, Kreislaufverdichter usw. können daher kleiner ausgestaltet werden oder es kann gegebenenfalls ganz auf sie verzichtet werden. Hierdurch reduzieren sich die entsprechenden Investitionskosten.

Durch die Optimierung des Reaktionstemperaturprofils entlang der Längskoordinate des Reaktors wird ferner die Bildung unerwünschter Nebenprodukte reduziert, wodurch ein reineres Zielprodukt erhalten wird und sich der Reinigungsaufwand verringert.

### Weitere besondere Ausgestaltungen der Erfindung

In einem zweiten Aspekt der Erfindung ist der erfindungsgemäße Reaktor dadurch gekennzeichnet, dass der erste gekühlte Abschnitt des mindestens einen Rohrreaktors oder Plattenreaktors den letzten Abschnitt der Katalysatorschüttung in Strömungsrichtung des Einsatzgemischstroms und des Produktgemischstroms umfasst. Der erste gekühlte Abschnitt des Reaktors fällt dabei mit dem letzten Abschnitt der Katalysatorschüttung zusammen. Da in den Innenraum dieses Abschnitts eingeleitete Kühlmittel mit der Kühlmittel-Kühlvorrichtung relativ zu seinem Siedepunkt unterkühlt wurde, liegt es hier flüssig vor, so dass Kühltemperaturen unterhalb des Siedepunktes eingestellt werden können. Aufgrund der hierdurch erreichten Temperaturabsenkung im Katalysatorbett wird es somit ermöglicht, das Reaktionsgleichgewicht in diesem Teil des Katalysatorbetts in Richtung auf die Reaktionsprodukte zu verschieben. Hierdurch wird eine zusätzliche Bildung von Reaktionsprodukten selbst für den Fall ermöglicht, dass das Reaktionsgleichgewicht zuvor, d. h. in einen ersten Teil des Katalysatorbetts, bereits erreicht wurde.

In einem dritten Aspekt der Erfindung ist der erfindungsgemäße Reaktor dadurch gekennzeichnet, dass der letzte Abschnitt der Katalysatorschüttung zwischen 0 und 30 % der Gesamtlänge der Katalysatorschüttung, bevorzugt zwischen 0 und 40 % der Gesamtlänge der Katalysatorschüttung, meist bevorzugt zwischen 0 und 50 % der Gesamtlänge der Katalysatorschüttung beträgt. Untersuchungen haben ergeben, dass bei entsprechender Dimensionierung des ersten gekühlte Abschnitt des Reaktors besonders günstige Produktausbeuten erhalten werden. Wird dagegen der erste gekühlte Abschnitt des Reaktors noch größer dimensioniert, wird der zweite gekühlte Abschnitt des Reaktors unter Umständen zu klein, so dass die hier erreichten Eduktumsätze bzw. Produktausbeuten zu gering sind und bei Bilanzierung über den gesamten Reaktor zu wenig Reaktionsprodukt erzeugt wird.

In einem vierten Aspekt der Erfindung ist der erfindungsgemäße Reaktor dadurch gekennzeichnet, dass die Kühlmittel-Kühlvorrichtung so einstellbar ist, dass die Temperatur des Kühlmittelstroms vor dem Eintritt in den Innenraum um mindestens 3 °C, bevorzugt um mindestens 10 °C, meist bevorzugt um mindestens 20 °C unterhalb der Siedetemperatur des Kühlmittels bei dem Kühlmitteldruck liegt. Untersuchungen haben ergeben, dass bei entsprechender Wahl der Unterkühlungstemperaturen im ersten gekühlten Abschnitt des Reaktors besonders günstige Produktausbeuten erhalten werden.

In einem fünften Aspekt der Erfindung ist der erfindungsgemäße Reaktor dadurch gekennzeichnet, dass der Reaktor eine Vielzahl von mit Katalysatorschüttungen gefüllten Rohrreaktor oder Plattenreaktor umfasst, die bezüglich ihrer Längsachse parallel in dem Mantelrohr angeordnet sind. Durch die Vervielfachung der Reaktorrohre in dem Kühlmantel multipliziert sich die Produktausbeute entsprechend.

In einem sechsten Aspekt der Erfindung ist der erfindungsgemäße Reaktor dadurch gekennzeichnet, dass der Reaktor bezüglich seiner Längsachse stehend angeordnet ist, so dass sich der Edukteinlass und der Kühlmittelauslass am oberen Ende des Reaktors befinden und sich der Produktauslass und der Kühlmitteleinlass am unteren Ende des Reaktors befinden. Dies ist besonders günstig, da hierdurch der zweite gekühlte Abschnitt an der Oberseite des Reaktors angeordnet ist. Da das Kühlmittel in diesem zweite gekühlten Abschnitt des Reaktors siedet und als Dampf oder Zweiphasenströmung aus dem Innenraum ausgeleitet wird, kann die Förderung des Kühlmittels mindestens teilweise im Naturumlauf erfolgen, so dass entsprechende Fördervorrichtungen, beispielsweise Kühlmittelpumpen, kleiner dimensioniert werden oder ganz entfallen können.

Ein siebter Aspekt der Erfindung betrifft die Verwendung eines Reaktors nach einem der oben genannten Aspekte für die Durchführung Methanolsynthese und/oder für die Ammon iaksynthese.

In einem neunten Aspekt der Erfindung ist das erfindungsgemäße Verfahren dadurch gekennzeichnet, dass der erste gekühlte Abschnitt des mindestens einen Rohrreaktors oder Plattenreaktors den letzten Abschnitt der Katalysatorschüttung in Strömungsrichtung des Einsatzgemischstroms und des Produktgemischstroms umfasst. Die mit dieser Ausgestaltung des Verfahrens verbundenen Vorteile entsprechen denen, die im Zusammenhang mit dem zweiten Aspekt der Erfindung bei dem erfindungsgemäßen Reaktor erörtert wurden.

In einem zehnten Aspekt der Erfindung ist das erfindungsgemäße Verfahren dadurch gekennzeichnet, dass der letzte Abschnitt der Katalysatorschüttung zwischen 0 und 30 % der Gesamtlänge der Katalysatorschüttung, bevorzugt zwischen 0 und 40 % der Gesamtlänge der Katalysatorschüttung, meist bevorzugt zwischen 0 und 50 % der Gesamtlänge der Katalysatorschüttung beträgt. Die mit dieser Ausgestaltung des Verfahrens verbundenen Vorteile entsprechen denen, die im Zusammenhang mit dem dritten Aspekt der Erfindung bei dem erfindungsgemäßen Reaktor erörtert wurden.

In einem elften Aspekt der Erfindung ist das erfindungsgemäße Verfahren dadurch gekennzeichnet, dass die Kühlmittel-Kühlvorrichtung so einstellbar ist, dass die Temperatur des Kühlmittelstroms vor dem Eintritt in den Innenraum um mindestens 3 °C, bevorzugt um mindestens 10 °C, meist bevorzugt um mindestens 20 °C unterhalb der Siedetemperatur des Kühlmittels bei dem Kühlmitteldruck liegt. Die mit dieser Ausgestaltung des Verfahrens verbundenen Vorteile entsprechen denen, die im Zusammenhang mit dem vierten Aspekt der Erfindung bei dem erfindungsgemäßen Reaktor erörtert wurden.

In einem zwölften Aspekt der Erfindung ist das erfindungsgemäße Verfahren dadurch gekennzeichnet, dass der Reaktor eine Vielzahl von mit Katalysatorschüttungen gefüllten Rohrreaktoren oder Plattenreaktoren umfasst, die bezüglich ihrer Längsachse parallel in dem Mantelrohr angeordnet sind. Die mit dieser Ausgestaltung des Verfahrens verbundenen Vorteile entsprechen denen, die im Zusammenhang mit dem fünften Aspekt der Erfindung bei dem erfindungsgemäßen Reaktor erörtert wurden.

In einem dreizehnten Aspekt der Erfindung ist das erfindungsgemäße Verfahren dadurch gekennzeichnet, dass der Reaktor bezüglich seiner Längsachse stehend angeordnet ist, so dass sich der Edukteinlass und der Kühlmittelauslass am oberen Ende des Reaktors befinden und sich der Produktauslass und der Kühlmitteleinlass am unteren Ende des Reaktors befinden. Die mit dieser Ausgestaltung des Verfahrens verbundenen Vorteile entsprechen denen, die im Zusammenhang mit dem sechsten Aspekt der Erfindung bei dem erfindungsgemäßen Reaktor erörtert wurden.

In einem vierzehnten Aspekt der Erfindung ist das erfindungsgemäße Verfahren dadurch gekennzeichnet, dass die Schritte (2) bis (5) wie folgt ausgestaltet sind:
(2) Bereitstellen eines gasförmigen Einsatzgemischstroms, der Wasserstoff und Kohlenstoffoxide als Eduktkomponenten enthält;
(3) Einleiten des gasförmigen Einsatzgemischstroms über den Edukteinlass in den mindestens einen Rohrreaktor oder Plattenreaktor;
(4) mindestens teilweises Umsetzen des gasförmigen Einsatzgemischstroms unter Methanolsynthesebedingungen zu einem fluiden Produktgemischstrom, der Methanol als Produktkomponente und nicht umgesetzte Eduktkomponenten enthält;
(5) Ausleiten des fluiden Produktgemischstroms über den Produktauslass aus dem mindestens einen Rohrreaktor oder Plattenreaktor.

Auf diese Weise wird ein vorteilhaftes Verfahren zur Methanolsynthese mit dem erfindungsgemäßen Reaktor erhalten.

In einem fünfzehnten Aspekt der Erfindung ist das erfindungsgemäße Verfahren dadurch gekennzeichnet, dass die Schritte (2) bis (5) wie folgt ausgestaltet sind:
(2) Bereitstellen eines gasförmigen Einsatzgemischstroms, der Wasserstoff und Stickstoff als Eduktkomponenten enthält;
(3) Einleiten des gasförmigen Einsatzgemischstroms über den Edukteinlass in den mindestens einen Rohrreaktor oder Plattenreaktor;
(4) mindestens teilweises Umsetzen des gasförmigen Einsatzgemischstroms unter Ammoniaksynthesebedingungen zu einem gasförmigen Produktgemischstrom, der Ammoniak als Produktkomponente und nicht umgesetzte Eduktkomponenten enthält;
(5) Ausleiten des gasförmigen Produktgemischstroms über den Produktauslass aus dem mindestens einen Rohrreaktor oder Plattenreaktor.

Auf diese Weise wird ein vorteilhaftes Verfahren zur Ammoniaksynthese mit dem erfindungsgemäßen Reaktor erhalten.

### Ausführungs- und Zahlenbeispiele

Weiterbildungen, Vorteile und Anwendungsmöglichkeiten der Erfindung ergeben sich auch aus der nachfolgenden Beschreibung von Ausführungs- und Zahlenbeispielen und den Zeichnungen. Dabei bilden alle beschriebenen und/oder bildlich dargestellten Merkmale für sich oder in beliebiger Kombination die Erfindung, unabhängig von ihrer Zusammenfassung in den Ansprüchen oder deren Rückbeziehung.

Es zeigt:
- Fig. 1: eine schematische Darstellung einer Anlage bzw. eines Verfahrens mit dem erfindungsgemäßen Reaktor;
- Fig. 2: ein axiales Temperaturprofil im Reaktor gemäß einer ersten Ausführungsform der Erfindung;
- Fig. 3: ein axiales Temperaturprofil im Reaktor gemäß einer zweiten Ausführungsform der Erfindung;
- Fig. 4: ein axiales Temperaturprofil im Reaktor gemäß einer dritten Ausführungsform der Erfindung.

Im Folgenden ist die Angabe "nicht gezeigt" so zu verstehen, dass ein Element in der besprochenen Abbildung nicht grafisch dargestellt, aber gemäß der Beschreibung trotzdem vorhanden ist.

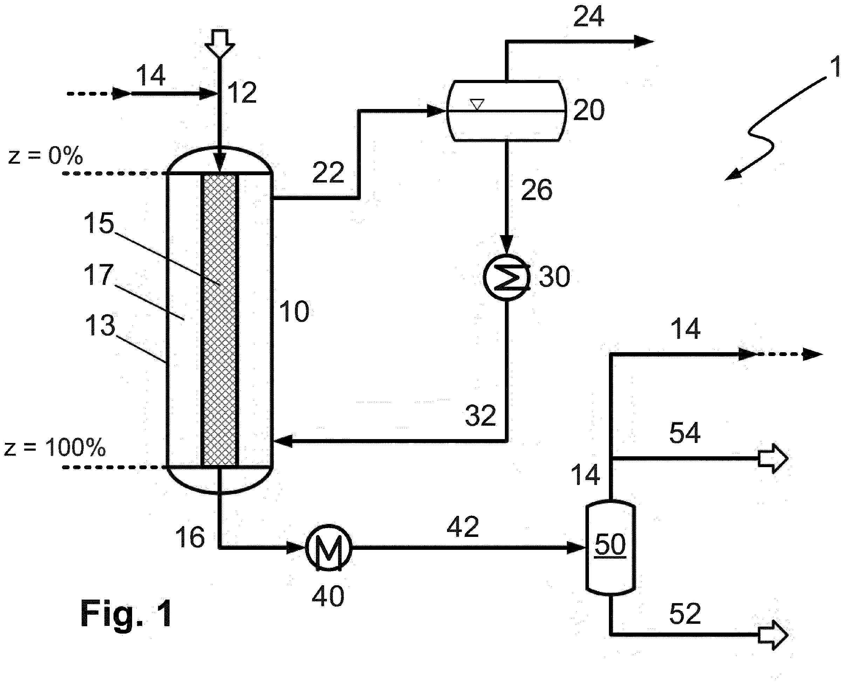

Fig. 1 zeigt ein Ausführungsbeispiel einer Anlage 1 bzw. eines Verfahrens 1 zur Methanolsynthese mit einem erfindungsgemäßen Reaktor 10.

Über eine Leitung 12 wird ein Frisch-Synthesegasstrom (Makeup-Gasstrom), enthaltend Wasserstoff und Kohlenoxide sowie Inertkomponenten wie beispielsweise Methan, in die Anlage bzw. in das Verfahren eingeführt und in einen im Inneren eines zylindrischen Mantelrohrs 13 angeordneten Rohrreaktor 15 über einen nicht gezeigten Edukteinlass eingeleitet. Zuvor wird ein Kreislaufgasstrom, der nicht umgesetzte Synthesegasbestandteile enthält, in einer Leitung 14 herangeführt und ebenfalls über Leitung 12 in den Rohrreaktor 15 eingeleitet. Sowohl das Frisch-Synthesegas als auch das Kreislaufgas werden vor dem Einleiten in den Rohrreaktor 15 mittels nicht gezeigter Verdichter auf den Reaktionsdruck verdichtet und gefördert.

Im Rohrreaktor 15 erfolgt ein Teilumsatz der Synthesegaskomponenten zu Methanol an einem Methanolsynthesekatalysator unter Methanolsynthesebedingungen, wobei wegen des exothermen Charakters der mit der Methanolsynthese verbundenen Reaktionen Wärme freigesetzt wird. Der Rohrreaktor 15 ist als zylindrischer Einzelrohrreaktor ausgebildet und mit einem für die Methanolsynthese aktiven Katalysator gefüllt. Die Längenkoordinate z des Katalysatorbetts ist in Fig. 1 angedeutet. Im vorliegenden Beispiel ist der Reaktor 10 stehend angeordnet.

Über eine Leitung 16 wird ein Produktgemischstrom, enthaltend Methanol, nicht umgesetzte Synthesegasbestandteile und Inertkomponenten, aus dem Rohrreaktor 15 und dem Reaktor 10 über einen nicht gezeigten Produktauslass ausgeleitet, mittels eines Kühlers 40 auf eine Temperatur unterhalb des Taupunktes abgekühlt und mittels einer Leitung 42 in eine Gas-Flüssig-Phasentrennvorrichtung 50 eingeleitet.

In der Gas-Flüssig-Phasentrennvorrichtung 50 erfolgt die Auftrennung des Produktgemischstroms in einen flüssigen Rohmethanolstrom, der über eine Leitung 52 aus der Gas-Flüssig-Phasentrennvorrichtung 50 ausgeleitet und einer nicht gezeigten Produktaufarbeitung zugeführt wird. Über Leitung 14 wird der größte Teil des nicht umgesetzten Synthesegas aus der Gas-Flüssig-Phasentrennvorrichtung 50 ausgeleitet und nach Verdichtung wiederum über Leitung 12 dem Reaktor 10 zugeführt. Über Leitung 14 und eine Leitung 54 wird ein kleinerer Teil des Synthesegases als Spülstrom oder Purgestrom aus der Anlage bzw. dem Verfahren ausgeleitet, um die Akkumulation von Inertkomponenten zu verhindern.

Der Rohrreaktor 15 wird mittels Wasser als Kühlmittel gekühlt, das als unterkühlter Kühlmittelstrom über eine Leitung 32 herangeführt und über einen nicht gezeigten Kühlmitteleinlass in den Innenraum 17 eingeleitet, der sich zwischen der Außenwand des Rohrreaktors 15 und der Innenwand des Mantelrohrs 13 befindet und im vorliegenden Beispiel eine Ringraumform aufweist.

Beim Durchströmen des Innenraums 17 im Gegenstrom zur Gasströmung der Eduktgase bzw. Produktgase nimmt das Kühlmittel einen Teil der durch die Methanolsynthese freigesetzten Reaktionswärme auf. Wegen der stehenden Anordnung des Reaktors 10 durchströmt das Kühlmittel den Innenraum 17 von unten nach oben. Wegen der Unterkühlung des Kühlmittels, die mittels eines Kühlers 30 bewirkt wird, bleibt es auf einem ersten gekühlten Abschnitt des Rohrreaktors, der dem bezüglich des Eduktstroms bzw. Produktstroms letzten Abschnitt des Katalysatorbetts zwischen der Längenkoordinate 100 % und einem Wert der Längenkoordinate z zwischen 0 und 100 % flüssig. Bei weiterem Durchströmen durch den Innenraum 17 beginnt das Kühlmittel zu sieden und verdampft ganz oder teilweise. Daher erfolgt die Kühlung des oberen Bereichs des Rohrreaktors 15 auf einem zweiten gekühlten Abschnitt des Rohrreaktors mittels dampfförmigem Kühlmittel oder als Zweiphasenströmung aus Dampf und Flüssigkeit. Der zweite gekühlte Abschnitt des Rohrreaktors 15 entspricht daher dem bezüglich des Eduktstroms bzw. Produktstroms ersten Abschnitt des Katalysatorbetts zwischen der Längenkoordinate 0 % und einem Wert der Längenkoordinate z zwischen 0 und 100 %.

Das verdampfte oder teilverdampfte Kühlmittel wird mittels einer Leitung 22 aus dem Reaktor 10 ausgeleitet und in ein als Dampftrommel ausgestaltetes Kühlmittel-Reservoir 20 eingeleitet. Über eine Leitung 24 wird dabei ein Teil des erzeugten Dampfs an Verbraucher abgegeben. Das somit dem Kühlkreislauf entzogene Kühlmittel wird durch Zuführen eines entsprechenden Wasserstroms als Kühlmittelstrom der Dampftrommel über eine nicht gezeigte Leitung zugeführt. Über eine Leitung 26 wird flüssiges, dampfgesättigtes Kühlmittel aus der Dampftrommel 20 ausgeleitet und dem Kühler 30 zugeführt. Im Kühler 30 wird eine definierte Unterkühlung des Kühlmittels bewirkt, das sodann über Leitung 32 wieder zum Reaktor 10 zurückgeführt wird.

### Zahlenbeispiele für die Methanolsynthese

**Tabelle 1: Wassergekühlter Reaktor, 2000 TPD Methanolproduktion. Reaktorvolumen 88.8 m³**

| **Makeup-Gas mit CO, CO₂**, **H₂; SN=2.13** | **SOR (6x4)** | | |
|---|---|---|---|
| | Vergleich 100% konstante Kühltemperatur; **Fig. 2** | Erfindung Letzte 30 % Kat.bett unterkühlt; **Fig. 3** | Erfindung Letzte 30 % Kat.bett unterkühlt; **Fig. 3** |
| MeOH/TPD | 2190 | 2206 | 2190 |
| X_CO2_pp | 0.32 | 0.35 | 0.33 |
| X CO_pp | 0.78 | 0.83 | 0.83 |
| RR | 3.2 | 3.2 | 2.85 |

### Erläuterungen:

- SN: Stöchiometriezahl Methanolsynthesegas
- SOR: Start of Run (Beginn Katalysatorzyklus)
- EOR: End of Run (Ende Katalysatorzyklus)
- 6x4, 3x3: Partikelgröße Katalysator
- TPD: (metrische) Tonnen pro Tag
- X_CO2_pp: Umsatz CO2 pro Reaktordurchgang
- X_CO_pp: Umsatz CO pro Reaktordurchgang
- RR: Rückführverhältnis nicht umgesetztes Synthesegas zum Reaktor
- LM: Layer Management, Schichten unterschiedlicher Katalysatoren

**Tabelle 2: Wassergekühlter Reaktor, 2000 TPD Methanol, Layer Management. Reaktorvolumen 88.8 m³**

| **Makeup-Gas mit CO, CO₂**, **H₂; SN=2.13** | **SOR** | | |
|---|---|---|---|
| | Vergleich 1 100% konstante Kühltemperatur; **Fig. 2** | Vergleich 2 100% konstante Kühltemperatur + LM (50% 6x4, 50% 3x3) | Erfindung Letzte 30 % Kat.bett unterkühlt + LM (50% 6x4, 50% 3x3); **Fig. 4** |
| MeOH/TPD | 2190 | 2197 | 2213 |
| X_CO2_pp | 0.32 | 0.33 | 0.38 |
| X_CO_pp | 0.78 | 0.80 | 0.86 |
| RR | 3.2 | 3.2 | 3.2 |

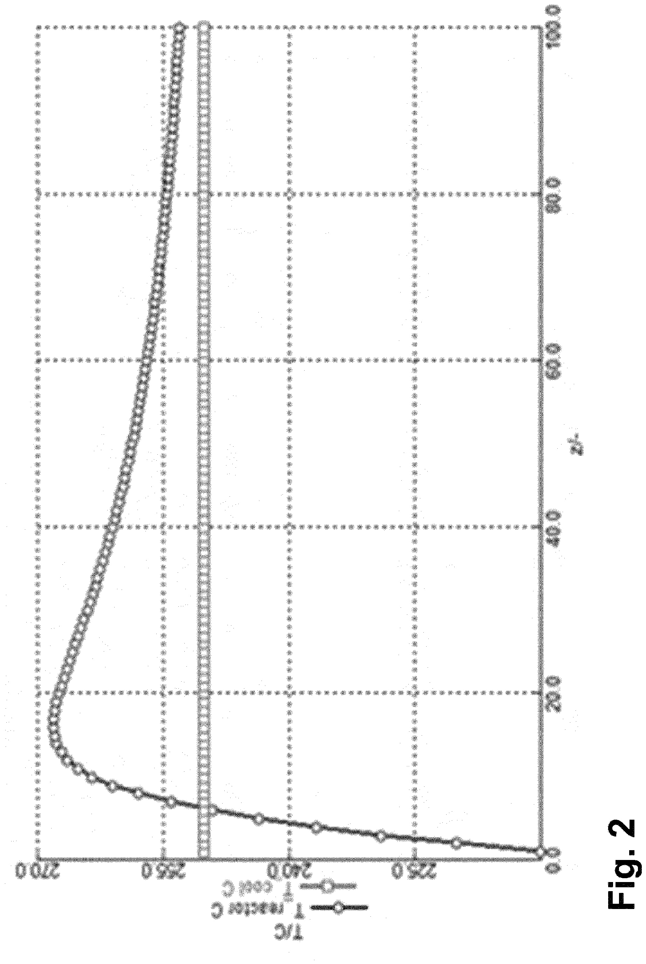

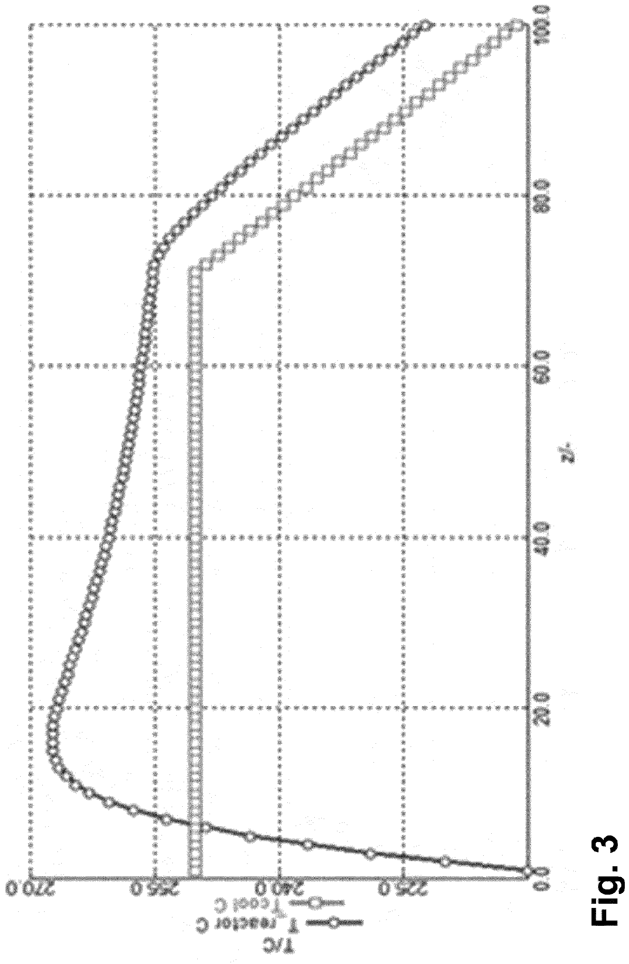

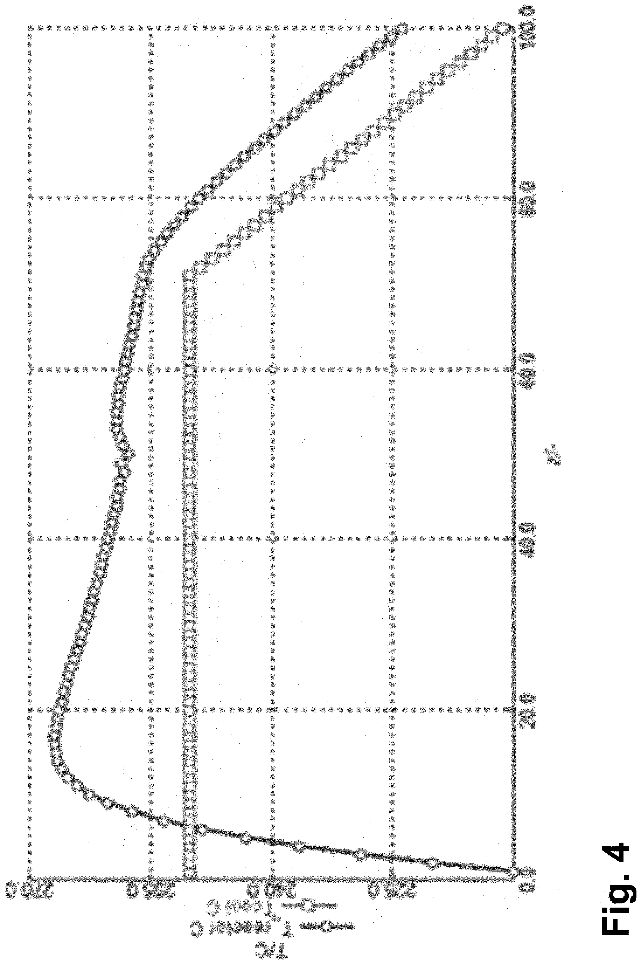

Wie anhand der in Tabelle 1 zusammengestellten Daten zu erkennen ist, erhöht sich die Methanolproduktion durch Unterkühlung der letzten 30 % des Katalysatorbetts von 2190 TPD auf 2206 TPD (SOR). Alternativ besteht mit der Erfindung die Möglichkeit, das Rückführverhältnis von 3.2 auf 2.85 abzusenken und trotzdem dieselbe Methanolproduktion zu erhalten. Die zugehörigen Temperaturprofile zeigt Fig. 2, wobei, wie auch in Fig. 3 und Fig. 4, runde Symbole Temperaturen im Reaktor (im Katalysatorbett) und eckige Symbole Temperaturen des Kühlmittels entsprechen.

Aus den in Tabelle 2 zusammengestellten Daten geht hervor, dass die Verwendung eines Katalysator Layer Managements, also der Verwendung von Schichten unterschiedlicher Katalysatoren, hier zweier Katalysatoren für die Methanolsynthese mit unterschiedlicher Partikelgröße, zusammen mit der erfindungsgemäßen Unterkühlung des Kühlmittels die Produktion bei konstantem Rückführverhältnis nochmals erhöht. Die zugehörigen Temperaturprofile zeigt Fig. 3.

**Tabelle 3: Wassergekühlter Reaktor, CO-reiches Synthesegas, 2000 TPD Methanol, Layer Management. Reaktorvolumen 88.8 m³**

| **Makeup-Gas mit CO, CO₂**, **H₂; SN=1.96** | **SOR** | | | | **EOR** | | | | |
|---|---|---|---|---|---|---|---|---|---|
| | Vergleich | Erfindung: Kühlung 40% | Erfindung: LM Kühlung 40% | Erfindung: LM Kühlung 40%+R R | Vergleich | Erfindung: Kühlung 40% | Erfindung: Kühlung 40% +RR | Erfindung: Kühlung 40% + LM | Erfindung: Kühlung 40% + LM + RR |
| MeOH/TPD | 2390 | 2406 | 2415 | 2390 | 2285 | 2302 | 2285 | 2348 | 2285 |
| X_CO2_pp | 0.075 | 0.077 | 0.08 | 0.09 | 0.061 | 0.056 | 0.060 | 0.062 | 0.068 |
| X_CO_pp | 0.55 | 0.605 | 0.64 | 0.75 | 0.38 | 0.41 | 0.50 | 0.47 | 0.65 |
| RR | 3.2 | 3.2 | 3.2 | 1.94 | 3.2 | 3.2 | 2.3 | 3.2 | 1.42 |

Aus den in Tabelle 3 zusammengestellten Daten ist ersichtlich, dass die im Zusammenhang mit Tabelle 1 und Tabelle 2 erläuterten Zusammenhänge nicht nur für SOR, sondern auch für EOR, und bei Durchführung des Verfahrens mit CI-reichem Synthesegas gelten. Die zugehörigen Temperaturprofile zeigt Fig. 4.

### Bezugszeichenliste

- [1]: Anlage bzw. Verfahren
- [10]: Reaktor
- [12]: Leitung
- [13]: Mantelrohr
- [14]: Leitung
- [15]: Rohrreaktor
- [16]: Leitung
- [17]: Innenraum
- [20]: Kühlmittel-Reservoir (Dampftrommel)
- [22]: Leitung
- [24]: Leitung
- [26]: Leitung
- [30]: Kühler (Wärmetauscher oder Luftkühler)
- [32]: Leitung
- [40]: Kühler
- [42]: Leitung
- [50]: Gas-Flüssig-Phasentrennvorrichtung
- [52]: Leitung
- [54]: Leitung

## Patentansprüche

1. Reaktor zum Durchführen exothermer Gleichgewichtsreaktionen, bei denen ein fluider Einsatzgemischstrom an einem festen Katalysator mindestens teilweise zu einem fluiden Produktgemischstrom umgesetzt wird, wobei der Reaktor die folgenden, in Fluidverbindung miteinander stehenden Bestandteile umfasst:
(a) mindestens einen Rohrreaktor oder Plattenreaktor, in dem ein Katalysator als fluiddurchlässige Katalysatorschüttung von festen Katalysatorpartikeln angeordnet ist, wobei der Katalysator aktiv für die Durchführung der exothermen Gleichgewichtsreaktion in einem Reaktionstemperaturbereich bei einem Reaktionsdruck ist;
(b) wobei der mindestens eine Rohrreaktor oder Plattenreaktor den Reaktionsdruck trägt und an seinem einen Ende einen Edukteinlass für den fluiden Einsatzgemischstrom und an seinem anderen Ende einen Produktauslass für den fluiden Produktgemischstrom aufweist;
(c) ein Mantelrohr, das den mindestens einen Rohrreaktor oder Plattenreaktor umgibt und dabei an seinen Enden fluiddicht gegenüber dem mindestens einen Rohrreaktor oder Plattenreaktor abgeschlossen ist, wobei das Mantelrohr einen Kühlmitteleinlass und einen Kühlmittelauslass für einen fluiden Kühlmittelstrom an entgegengesetzten Enden des Mantelrohrs aufweist;
(d) wobei der Innenraum zwischen der Außenwand des mindestens einen Rohrreaktors oder den Außenwänden des mindestens einen Plattenreaktors und der Innenwand des Mantelrohrs von dem Kühlmittelstrom durchströmbar ist, wobei der Kühlmitteleinlass und der Kühlmittelauslass so angeordnet sind, dass der Kühlmittelstrom den Reaktor im Gegenstrom zu dem Einsatzgemischstrom und dem Produktgemischstrom durchläuft;
(e) ein Kühlmittel zur Erzeugung des Kühlmittelstroms, wobei das Kühlmittel so ausgewählt wird, dass es bei einem festgelegten Kühlmitteldruck in dem Reaktionstemperaturbereich verdampft;
(f) eine Druckregelvorrichtung zum Einstellen des Kühlmitteldrucks im Innenraum;
(g) eine Kühlmittel-Kühlvorrichtung, die es ermöglicht, dass der Kühlmittelstrom im flüssigen Zustand über den Kühlmitteleinlass in den Innenraum eintritt, den Innenraum zunächst als Flüssigkeit durchströmt und dabei einen ersten gekühlten Abschnitt des mindestens einen Rohrreaktors oder Plattenreaktors kühlt, sodann verdampft und im dampfförmigen Zustand und/oder als Zweiphasenströmung aus flüssigem und dampfförmigem Kühlmittel den restlichen, zweiten gekühlten Abschnitt des mindestens einen Rohrreaktor oder Plattenreaktor kühlt und sodann im dampfförmigen Zustand und/oder als Zweiphasenströmung aus flüssigem und dampfförmigem Kühlmittel über den Kühlmittelauslass aus dem Innenraum ausgeleitet wird.

2. Reaktor nach Anspruch 1, **dadurch gekennzeichnet, dass** der erste gekühlte Abschnitt des mindestens einen Rohrreaktors oder Plattenreaktors den letzten Abschnitt der Katalysatorschüttung in Strömungsrichtung des Einsatzgemischstroms und des Produktgemischstroms umfasst.

3. Reaktor nach Anspruch 1 oder 2, **dadurch gekennzeichnet, dass** der letzte Abschnitt der Katalysatorschüttung zwischen 0 und 30 % der Gesamtlänge der Katalysatorschüttung, bevorzugt zwischen 0 und 40 % der Gesamtlänge der Katalysatorschüttung, meist bevorzugt zwischen 0 und 50 % der Gesamtlänge der Katalysatorschüttung beträgt.

4. Reaktor nach einem der Ansprüche 1 bis 3, **dadurch gekennzeichnet, dass** die Kühlmittel-Kühlvorrichtung so einstellbar ist, dass die Temperatur des Kühlmittelstroms vor dem Eintritt in den Innenraum um mindestens 3 °C, bevorzugt um mindestens 10 °C, meist bevorzugt um mindestens 20 °C unterhalb der Siedetemperatur des Kühlmittels bei dem Kühlmitteldruck liegt.

5. Reaktor nach einem der Ansprüche 1 bis 4, **dadurch gekennzeichnet, dass** der Reaktor eine Vielzahl von mit Katalysatorschüttungen gefüllten Rohrreaktoren oder Plattenreaktoren umfasst, die bezüglich ihrer Längsachse parallel in dem Mantelrohr angeordnet sind.

6. Reaktor nach einem der Ansprüche 1 bis 5, **dadurch gekennzeichnet, dass** der Reaktor bezüglich seiner Längsachse Kühlmittel-Kühlvorrichtung, angeordnet ist, so dass sich der Edukteinlass und der Kühlmittelauslass am oberen Ende des Reaktors befinden und sich der Produktauslass und der Kühlmitteleinlass am unteren Ende des Reaktors befinden.

7. Verwendung eines Reaktors nach einem der Ansprüche 1 bis 6 für die Methanolsynthese und/oder für die Ammoniaksynthese.

8. Verfahren zum Durchführen exothermer Gleichgewichtsreaktionen, bei denen ein fluider Einsatzgemischstrom an einem festen Katalysator mindestens teilweise zu einem fluiden Produktgemischstrom umgesetzt wird, wobei das Verfahren folgende Schritte umfasst:
(1) Bereitstellen eines Reaktors, umfassend:
(a) mindestens einen Rohrreaktor oder Plattenreaktor, in dem ein Katalysator als fluiddurchlässige Katalysatorschüttung von festen Katalysatorpartikeln angeordnet ist, wobei der Katalysator aktiv für die Durchführung der exothermen Gleichgewichtsreaktion in einem Reaktionstemperaturbereich bei einem Reaktionsdruck ist;
(b) wobei der Rohrreaktor oder Plattenreaktor den Reaktionsdruck trägt und an seinem einen Ende einen Edukteinlass für den fluiden Einsatzgemischstrom und an seinem anderen Ende einen Produktauslass für den fluiden Produktgemischstrom aufweist;
(c) ein Mantelrohr, das den mindestens einen Rohrreaktor oder Plattenreaktor umgibt und dabei an seinen Enden fluiddicht gegenüber dem mindestens einen Rohrreaktor oder Plattenreaktor abgeschlossen ist, wobei das Mantelrohr einen Kühlmitteleinlass und einen Kühlmittelauslass für einen fluiden Kühlmittelstrom an entgegengesetzten Enden des Mantelrohrs aufweist;
(d) wobei der Innenraum zwischen der Außenwand des mindestens einen Rohrreaktors oder den Außenwänden des mindestens einen Plattenreaktors und der Innenwand des Mantelrohrs von dem Kühlmittelstrom durchströmbar ist, wobei der Kühlmitteleinlass und der Kühlmittelauslass so angeordnet sind, dass der Kühlmittelstrom den Reaktor im Gegenstrom zu dem Einsatzgemischstrom und dem Produktgemischstrom durchläuft;
(e) ein Kühlmittel zur Erzeugung des Kühlmittelstroms, wobei das Kühlmittel so ausgewählt wird, dass es bei einem festgelegten Kühlmitteldruck in dem Reaktionstemperaturbereich verdampft;
(f) eine Druckregelvorrichtung zum Einstellen des Kühlmitteldrucks im Innenraum;
(g) eine Kühlmittel-Kühlvorrichtung, die es ermöglicht, dass der Kühlmittelstrom im flüssigen Zustand über den Kühlmitteleinlass in den Innenraum eintritt, den Innenraum zunächst als Flüssigkeit durchströmt und dabei einen ersten gekühlten Abschnitt des mindestens einen Rohrreaktors oder Plattenreaktors kühlt, sodann verdampft und im dampfförmigen Zustand und/oder als Zweiphasenströmung aus flüssigem und dampfförmigem Kühlmittel den restlichen, zweiten gekühlten Abschnitt des mindestens einen Reaktorrohr kühlt und sodann im dampfförmigen Zustand und/oder als Zweiphasenströmung aus flüssigem und dampfförmigem Kühlmittel über den Kühlmittelauslass aus dem Innenraum ausgeleitet wird;
(2) Bereitstellen eines fluiden Einsatzgemischstroms, der Eduktkomponenten enthält;
(3) Einleiten des fluiden Einsatzgemischstroms über den Edukteinlass in den mindestens einen Rohrreaktor oder Plattenreaktor;
(4) Mindestens teilweises Umsetzen des gasförmigen Einsatzgemischstroms unter Bedingungen der exothermen Gleichgewichtsreaktion zu einem fluiden Produktgemischstrom, der Produktkomponenten und nicht umgesetzte Eduktkomponenten enthält;
(5) Ausleiten des fluiden Produktgemischstroms über den Produktauslass aus dem mindestens einen Rohrreaktor oder Plattenreaktor.

9. Verfahren nach Anspruch 8, **dadurch gekennzeichnet, dass** der erste gekühlte Abschnitt des mindestens einen Rohrreaktors oder Plattenreaktors den letzten Abschnitt der Katalysatorschüttung in Strömungsrichtung des Einsatzgemischstroms und des Produktgemischstroms umfasst.

10. Verfahren nach Anspruch 8 oder 9, **dadurch gekennzeichnet, dass** der letzte Abschnitt der Katalysatorschüttung zwischen 0 und 30 % der Gesamtlänge der Katalysatorschüttung, bevorzugt zwischen 0 und 40 % der Gesamtlänge der Katalysatorschüttung, meist bevorzugt zwischen 0 und 50 % der Gesamtlänge der Katalysatorschüttung beträgt.

11. Verfahren nach einem der Ansprüche 8 bis 10, **dadurch gekennzeichnet, dass** die Kühlmittel-Kühlvorrichtung so einstellbar ist, dass die Temperatur des Kühlmittelstroms vor dem Eintritt in den Innenraum um mindestens 3 °C, bevorzugt um mindestens 10 °C, meist bevorzugt um mindestens 20 °C unterhalb der Siedetemperatur des Kühlmittels bei dem Kühlmitteldruck liegt.

12. Verfahren nach einem der Ansprüche 8 bis 11, **dadurch gekennzeichnet, dass** der Reaktor eine Vielzahl von mit Katalysatorschüttungen gefüllten Rohrreaktoren oder Plattenreaktoren umfasst, die bezüglich ihrer Längsachse parallel in dem Mantelrohr angeordnet sind.

13. Verfahren nach einem der Ansprüche 8 bis 12, **dadurch gekennzeichnet, dass** der Reaktor bezüglich seiner Längsachse stehend angeordnet ist, so dass sich der Edukteinlass und der Kühlmittelauslass am oberen Ende des Reaktors befinden und sich der Produktauslass und der Kühlmitteleinlass am unteren Ende des Reaktors befinden.

14. Verfahren nach einem der Ansprüche 8 bis 13, **dadurch gekennzeichnet, dass** die Schritte (2) bis (5) wie folgt ausgestaltet sind:
(2) Bereitstellen eines gasförmigen Einsatzgemischstroms, der Wasserstoff und Kohlenstoffoxide als Eduktkomponenten enthält;
(3) Einleiten des gasförmigen Einsatzgemischstroms über den Edukteinlass in den mindestens einen Rohrreaktor oder Plattenreaktor;
(4) mindestens teilweises Umsetzen des gasförmigen Einsatzgemischstroms unter Methanolsynthesebedingungen zu einem fluiden Produktgemischstrom, der Methanol als Produktkomponente und nicht umgesetzte Eduktkomponenten enthält;
(5) Ausleiten des fluiden Produktgemischstroms über den Produktauslass aus dem mindestens einen Rohrreaktor oder Plattenreaktor.

15. Verfahren nach einem der Ansprüche 8 bis 13, **dadurch gekennzeichnet, dass** die Schritte (2) bis (5) wie folgt ausgestaltet sind:
(2) Bereitstellen eines gasförmigen Einsatzgemischstroms, der Wasserstoff und Stickstoff als Eduktkomponenten enthält;
(3) Einleiten des gasförmigen Einsatzgemischstroms über den Edukteinlass in den mindestens einen Rohrreaktor oder Plattenreaktor;
(4) mindestens teilweises Umsetzen des gasförmigen Einsatzgemischstroms unter Ammoniaksynthesebedingungen zu einem gasförmigen Produktgemischstrom, der Ammoniak als Produktkomponente und nicht umgesetzte Eduktkomponenten enthält;
(5) Ausleiten des gasförmigen Produktgemischstroms über den Produktauslass aus dem mindestens einen Rohrreaktor oder Plattenreaktor.
